(19) Europäisches Patentamt
European Patent Office
Office européen des brevets

(11) EP 0 819 673 A1

(12) **EUROPÄISCHE PATENTANMELDUNG**

(43) Veröffentlichungstag:
**21.01.1998 Patentblatt 1998/04**

(21) Anmeldenummer: **97111942.5**

(22) Anmeldetag: **14.07.1997**

(51) Int. Cl.[6]: **C07C 261/04**, C01C 3/16, C07D 295/20, D06L 3/02, C11D 3/39

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **19.07.1996 DE 19629162**

(71) Anmelder: **Clariant GmbH**
**65929 Frankfurt am Main (DE)**

(72) Erfinder:
- **Löffler, Matthias, Dr.**
  **65527 Niedernhausen (DE)**
- **Reinhardt, Gerd, Dr.**
  **65779 Kelkheim (DE)**

(54) **Salze von Cyanamiden als Bleichaktivatoren**

(57) Verbindungen der allgemeinen Formel

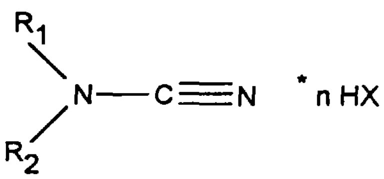

werden beansprucht, worin die Reste $R_1$ und $R_2$ Wasserstoff oder organische Substituenten, n entweder 1 oder 2 und X ein geeignetes Anion bedeuten. Diese Verbindungen werden als Bleichaktivatoren in bleichenden Wasch- und Reinigungsmittel eingesetzt.

EP 0 819 673 A1

## Beschreibung

Diese Erfindung betrifft die Verwendung Von Salzen von Cyanamiden als Bleichaktivatoren und Waschmittelzusammensetzungen, die diese Verbindungen als Bleichaktivatoren enthalten.

Es ist bekannt, daß das Bleichvermögen peroxidischer Bleichmittel, wie Perborate, Percarbonate, Persilicate und Perphosphate, verbessert werden kann, so daß die Bleichwirkung bei niedrigeren Temperaturen einsetzt, etwa bei oder unter 60°C, indem man die Vorstufen von bleichenden Peroxysäuren zusetzt, die oft als Bleichaktivatoren bezeichnet werden.

Viele Substanzen sind nach dem Stand der Technik als Bleichaktivatoren bekannt. Gewöhnlich handelt es sich dabei um reaktive organische Verbindungen mit einer O-Acyl- oder N-Acyl-Gruppe, die in alkalischer Lösung zusammen mit einer Quelle für Wasserstoffperoxid die entsprechenden Peroxysäuren bilden.

Repräsentative Beispiele für Bleichaktivatoren sind etwa N,N,N,N-Tetraacetylethylendiamin (TAED), Glucosepentaacetat (GPA), Xylosetetraacetat (TAX), Natrium-4-benzoyloxybenzolsulfonat (SBOBS), Natriumtrimethylhexanoyloxybenzolsulfonat (STHOBS), Tetraacetylglucoluril (TAGU), Tetraacetylcyansäure (TACA), Di-N-acetyldimethylglyoxin (ADMG) und 1-Phenyl-3-acetylhydantoin (PAH). Es sei beispielsweise auf GB-A-836 988, GB-A-907 356, EP-A-0 098 129 und EP-A-0 120 591 verwiesen.

Mittlerweile haben Verbindungen, die z.B. eine Nitrilgruppe enthalten, an Bedeutung gewonnen, da sie hocheffektive Bleichaktivatoren sind. Solche nitrilischen Bleichaktivatoren sind beispielsweise in EP 8475, GB 802 035, US 3 986 973, DD 286 182 und DE-A-2 407 906, beschrieben.

Wahrscheinlich bilden diese Verbindungen bei der Perhydrolyse eine Peroxyimidsäure, welche das bleichende Agens ist.

Nitrile der Formel

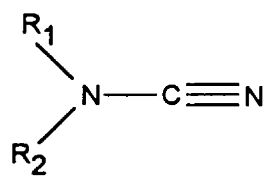

bilden dabei eine besondere Klasse nitrilischer Bleichaktivatoren. Cyanamide dieser Art und deren Verwendung als Bleichaktivatoren in Bleichmitteln sind beschrieben z.B. in EP 8475, US 4 025 453, US 4 086 175, US 4 086 177, DE-A-2 047 289. Bei allen dort beschriebenen Verbindungen handelt es sich um unsubstituierte und substituierte Cyanamide oder deren Metallsalze, wie sie durch Zugabe von Basen erhalten werden. Nachteile bei der Verwendung der beschriebenen Bleichaktivatoren sind u.a. starke Farb- und Faserschädigung am Waschgut, so daß sich diese Verbindungen als Bleichmittel lediglich in der Papierindustrie durchsetzen konnten.

Überraschenderweise wurde nun gefunden, daß Säureadditionssalze von Cyanamiden der zuvor beschriebenen Art, wie sie durch Zugabe von Säuren erhalten werden, einerseits über eine sehr gute Bleichleistung verfügen, andererseits aber keine Farb- und Faserschäden verursachen. Dies stellt einen Vorteil dar gegenüber den Cyanamid-Derivaten gemäß dem Stand der Technik. Die beschriebenen Verbindungen liegen als Reinsubstanz in Form eines farblosen Feststoffes vor und sind daher für den bestimmungsgemäßen Gebrauch besonders einfach zu handhaben.

Gegenstand der Erfindung sind somit Verbindungen der allgemeinen Formel

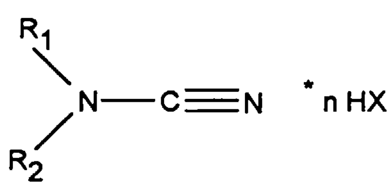

worin

$R_1$ und $R_2$ Wasserstoff, $C_1$- bis $C_{24}$-Alkyl, $C_2$-$C_{24}$-Alkenyl, Aryl, $C_1$-$C_4$-Alkylaryl oder $C_1$-$C_4$-Alkoxy-$C_1$-$C_4$-Alkyl sind, oder $R_1$ und $R_2$ zusammen mit dem Stickstoffatom, an das sie gebunden sind, einen Ring mit 4 bis 6 Kohlenstoffatomen bilden, der durch $C_1$-$C_5$-Alkyl, $C_1$-$C_5$-Alkoxy, $C_1$-$C_5$-Alkanoyl, Phenyl, Amino, Ammonium Cyano,

Cyanamino, Chlor oder Brom substituiert sein kann, und wobei dieser Ring zusätzlich zum Stickstoff anstelle von Kohlenstoffatomen ein oder zwei Sauerstoffatome oder >NH oder >N-CN-Gruppen enthalten kann,

n 1 oder 2 ist und X ein Anion bedeutet.

Die Reste $R_1$ und $R_2$ können substituiert oder unsubstituiert sein. Vorzugsweise bedeuten $R_1$ und $R_2$ unabhängig voneinander Wasserstoff, $C_1$-$C_4$-Alkyl, $C_2$-$C_4$-Alkenyl oder Phenyl. Als Anion X kommt beispielsweise Chlorid, Bromid, Iodid, Fluorid, Sulfat, Hydrogensulfat, Carbonat, Hydrogencarbonat, Phosphat, Mono- und Di-Hydrogenphosphat, Pyrophosphat, Metaphosphat, Nitrat, Methosulfat, Dodecylsulfat, Dodecylbenzolsulfonat, Tosylat, Phosphonat, Methylphosphonat, Methandisulfonat, Methylsulfonat, Ethansulfonat, Formiat, Acetat oder Propionat in Frage.

Besonders bevorzugt sind Salze des unsubstituierten Cyanamids mit zwei äquivalenten Säuren, wie Cyanamid * 2 HCl oder Cyanamid * $H_2SO_4$ sowie die entsprechenden kurzkettigen substituierten Derivate wie Dimethylcyanamid * 2 HCl oder Dimethylcyanamid *$H_2SO_4$.

An Hand eines allgemeinen Beispiels sollen die Synthesewege zu den Cyanamid-Salzen dieser Erfindung dargestellt werden.

1. Beim Einleiten von trockenem Salzsäuregas in eine Lösung von Cyanamid in einem geeigneten Lösemittel wie wasserfreiem Diethylether erhält man das entsprechende Dihydrochlorid als Niederschlag in quantitativer Ausbeute. Das Einleiten des Salzsäuregases erfolgt dabei bei Temperaturen von -10 bis 30°C, vorzugsweise bei 10°C. Das Einleiten wird so lange fortgesetzt, bis die Lösung kein Gas mehr aufnimmt, das Ende der Reaktion ist nach wenigen Stunden erreicht.

2. Gut kristallisiert erhält man Cyanamiddihydrochlorid durch Lösen von Cyanamid in konz. Salzsäure und Eindunsten der Lösung im Vakuum.

Gegenstand der Erfindung sind auch bleichende Wasch- und Reinigungsmittel, die die erfindungsgemäßen Verbindungen enthalten. Diese Wasch- und Reinigungsmittel enthalten neben einer Peroxyverbindung und dem Bleichaktivator üblicherweise auch oberflächenaktive Verbindungen und weitere Inhaltsstoffe.

Geeignete Peroxyverbindungen sind Alkaliperoxide, organische Peroxide wie Harnstoffperoxid, und anorganische Persalze, wie die Alkaliperborate, -percarbonate, -perphosphate, -persilicate und -persulfate. Mischungen aus zwei oder mehreren dieser Verbindungen sind ebenfalls geeignet. Besonders bevorzugt sind Natriumperborat-Tetrahydrat und insbesondere Natriumperborat-Monohydrat. Natriumperborat-Monohydrat ist wegen seiner guten Lagerbeständigkeit und seiner guten Löslichkeit in Wasser bevorzugt. Natriumpercarbonat kann aus Umweltschutzgründen bevorzugt sein.

Alkylhydroperoxide sind eine weitere geeignete Gruppe von Peroxyverbindungen. Beispiele für diese Stoffe sind Cumolhydroperoxid und t-Butylhydroperoxid.

In derartigen Wasch- und Reinigungsmitteln kann der erfindungsgemäße Bleichaktivator mit einem Gewichtsanteil von etwa 0,1 bis 20 %, bevorzugt von 0,5 bis 10 %, insbesondere von 1 bis 7,5 % vorhanden sein, zusammen mit einer Peroxyverbindung. Der Gewichtsanteil dieser Peroxyverbindungen beträgt gewöhnlich von 2 bis 40 %, bevorzugt von 4 bis 30 %, insbesondere von 10 bis 25 %.

In den Wasch- und Reinigungsmitteln können neben den erfindungsgemäßen Bleichaktivatoren noch andere geeignete Bleichaktivatoren, wie beispielsweise TAED, Tetraacetylglykoluril, Glucosepentaacetat, Natriumnonaoyloxybenzolsulfonat, Benzolcaprolactam oder nitrilische Aktivatoren enthalten sein. Diese zusätzlichen Bleichaktivatoren können in einer Menge von 1 bis 10 Gew.-% vorhanden sein.

Die oberflächenaktive Substanz kann von Naturprodukten abgeleitet sein, wie etwa Seite, oder ist eine synthetische Verbindung aus der Gruppe der anionischen nichtionischen, amphoteren, zwitterionischen oder kationischen oberflächenaktiven Substanzen, oder Mischungen aus diesen. Viele geeignete Substanzen sind kommerziell erhältlich, und sind in der Literatur beschrieben, beispielsweise in "Surface active agents and detergents", Vol. 1 und 2, von Schwartz, Perry und Berch. Der Gesamtanteil der oberflächenaktiven Verbindungen kann bis zu 50 Gew.-% betragen, vorzugsweise 1 Gew.-% bis 40 Gew.-%, insbesondere 4 Gew.-% bis 25 Gew.-%.

Synthetische anionische oberflächenaktive Substanzen sind üblicherweise wasserlösliche Alkalimetallsalze organischer Sulfate und Sulfonate mit Alkylresten von etwa 8 bis 22 Kohlenstoffatomen, wobei der Ausdruck "Alkyl" die Alkylsubstituenten höherer Arylreste einschließt.

Beispiele geeigneter anionischer Detergentien sind Natrium- und Ammoniumalkylsulfate, speziell die durch Sulfatierung höherer ($C_8$ bis $C_{18}$) Alkohole erhaltenen Sulfate; Natrium- und Ammoniumalkylbenzolsulfonate mit einem Alkylrest von $C_9$ bis $C_{20}$, insbesondere lineare sekundäre Natriumalkylbenzolsulfonate mit einem Alkylrest von $C_{10}$ bis $C_{15}$; Natriumalkylglycerinethersulfate, besonders die Ester der höheren, von Talg- und Kokosnußöl abgeleiteten Alkohole; die Natriumsulfate und -sulfonate der Kokosfettsäuremonoglyceride; Natrium- und Ammoniumsalze der Schwefelsäureester höherer ($C_9$ bis $C_{18}$) oxalkylierter, insbesondere der mit Ethylenoxid oxalkylierten Fettalkohole; die

Reaktionsprodukte der Veresterung von Fettsäuren mit Isethionsäure und nachfolgender Neutralisierung mit Natriumhydroxid; Natrium- und Ammoniumsalzen der Fettsäureamide des Methyltaurins; Alkan-Monosulfonate wie diejenigen aus der Reaktion von α-Olefinen ($C_8$-$C_{20}$) mit Natriumbisulfit und diejenigen aus der Reaktion von Paraffinen mit $SO_2$ und $Cl_2$ mit anschließender basischer Hydrolyse, wobei ein Gemisch verschiedener Sulfonate entsteht; Natrium- und Ammoniumdialkylsulfosuccinate mit Alkylresten von $C_7$ bis $C_{12}$; und Olefinsulfonate, die bei der Reaktion von Olefinen, insbesondere $C_{10}$- bis $C_{20}$-α-Olefinen, mit $SO_3$ und nachfolgender Hydrolyse der Reaktionsprodukte entstehen. Die bevorzugten anionischen Detergentien sind Natriumalkylbenzolsulfonate mit Alkylresten von $C_{15}$ bis $C_{18}$, und Natriumalkylethersulfate mit Alkylresten von $C_{16}$ bis $C_{18}$.

Beispiele für geeignete nichtionische oberflächenaktive Verbindungen, die bevorzugt zusammen mit anionischen oberflächenaktiven Verbindungen benutzt werden, sind insbesondere die Reaktionsprodukte von Alkylenoxiden (gewöhnlich Ethylenoxid) mit Alkylphenolen (Alkylreste von $C_5$ bis $C_{22}$), wobei die Reaktionsprodukte im allgemeinen 5 bis 25 Ethylenoxid (EO)-Einheiten im Molekül enthalten; die Reaktionsprodukte aliphatischer ($C_8$ bis $C_{18}$) primärer oder sekundärer, linearer oder verzweigter Alkohole mit Ethylenoxid, mit im allgemeinen 6 bis 30 EO, und die Additionsprodukte von Ethylenoxid an Reaktionsprodukte aus Propylenoxid und Ethylendiamin. Andere nichtionische oberflächenaktive Verbindungen sind Alkylpolyglycoside, langkettige tertiäre Aminoxide, langkettige tertiäre Phosphinoxide und Dialkylsulfoxide.

Amphotere oder zwitterionische oberflächenaktive Verbindungen können ebenfalls in den erfindungsgemäßen Zusammensetzungen verwendet werden, was aber wegen deren hoher Kosten meistens nicht bevorzugt ist. Wenn amphotere oder zwitterionische Verbindungen verwendet werden, so geschieht das in der Regel in kleinen Mengen in Zusammensetzungen, die hauptsächlich anionische und nichtionische Tenside enthalten.

Auch Seifen können in den erfindungsgemäßen Zusammensetzungen verwendet werden, vorzugsweise mit einem Anteil von weniger als 25 Gew.-%. Sie sind besonders geeignet in geringen Mengen in binären (Seite/anionisches Tensid) oder in ternären Mischungen zusammen mit nichtionischen oder gemischten synthetischen anionischen und nichtionischen Tensiden. Die verwendeten Seifen sind bevorzugt die Natriumsalze, und weniger bevorzugt die Kaliumsalze gesättigter oder ungesättigter $C_{10}$- bis $C_{24}$-Fettsäuren oder deren Mischungen. Die Anteile solcher Seifen können von 0,5 bis 25 Gew.-% betragen, geringere Mengen von 0,5 bis 5 Gew.-% sind im allgemeinen ausreichend zur Schaumkontrolle. Seifenanteile zwischen 2 und etwa 20 %, besonders zwischen etwa 5 und etwa 10 %, haben einen positiven Effekt. Dieses ist besonders der Fall in hartem Wasser, wo die Seife als zusätzliche Buildersubstanz dient.

Die Wasch- und Reinigungsmittel enthalten im allgemeinen auch einen Builder. Als Builder kommen in Betracht: Calcium bindende Stoffe, Fällungsmittel, Calciumspezifische Ionenaustauscher und deren Mischungen. Beispiele für Calcium bindende Stoffe umfassen Alkalimetallpolyphosphate, wie Natriumpolyphosphat; Nitrilotriessigsäure und ihre wasserlöslichen Salze; die Alkalimetallsalze der Carboxymethyloxybernsteinsäure, Ethylendiamintetraessigsäure, Oxydibernsteinsäure, Mellithsäure, Benzolpolycarbonsäuren, Zitronensäure; und Polyacetalcarboxylate, wie in US-4 144 226 und US-4 146 495 offenbart.

Beispiele für Fällungsmittel sind Natriumorthophosphat, Natriumcarbonat und Seifen aus langkettigen Fettsäuren.

Beispiele für Ionentauscher, die für Calcium spezifisch sind, sind die verschiedenen Arten wasserunlöslicher, kristalliner oder amorpher Aluminiumsilicate, von denen die Zeolithe die bekanntesten Vertreter sind.

Diese Buildersubstanzen können von 5 bis 80 Gew.-% vorhanden sein, bevorzugt ist ein Anteil von 10 bis 60 Gew.-%.

Neben den bereits erwähnten Inhaltsstoffen können die Wasch- und Reinigungsmittel jeden der konventionellen Zusatzstoffe in Mengen enthalten, die man üblicherweise in solchen Mitteln vorfindet. Beispiele dieser Zusatzstoffe umfassen Schaumbildner, wie etwa Alkanolamide, besonders die Monoethanolamide aus Palmkernöl-Fettsäuren und Kokosnuß-Fettsäuren; schaumverhindernde Substanzen, wie etwa Alkylphosphate und -silicone; Vergrauungsinhibitoren und ähnliche Hilfsmittel, wie etwa Natriumcarboxymethylcellulose und Alkyl- oder substituierte Alkylcelluloseether; Stabilisatoren, wie Ethylendiamintetraessigsäure, Weichmacher für Textilien; anorganische Salze, wie Natriumsulfat; und, in üblicherweise kleinen Mengen, fluoreszierende Stoffe, Parfüme, Enzyme wie Proteasen, Cellulosen, Lipasen und Amylasen, Desinfektionsmittel und Farbstoffe. Die Bleichaktivatoren dieser Erfindung können in einer Vielzahl von Produkten eingesetzt werden. Diese umfassen Textilwaschmittel, Textilbleichmittel, Oberflächenreiniger, Toilettenreiniger, Geschirrspülmaschinenreiniger und auch Gebißreiniger. Die Waschmittel können in fester Form oder flüssiger Form vorliegen.

Es ist aus Gründen der Stabilität und Handhabbarkeit vorteilhaft, die Bleichaktivatoren in Form von Granulaten zu verwenden, die neben dem Bleichaktivator ein Bindemittel enthalten. Verschiedene Methoden, solche Granulate herzustellen, sind in der Patentliteratur beschrieben, so beispielsweise in CA-1 102 966, GB-1 561 333, US-4 087 369, EP-A-0 240 057, EP-A-0 241 962, EP-A-0 101 634 und EP-A-0 062 523. Jede dieser Methoden ist für die erfindungsgemäßen Bleichaktivatoren anwendbar.

Die die Bleichaktivatoren enthaltenden Granulate werden im allgemeinen der Waschmittelzusammensetzung zusammen mit den anderen, trockenen Bestandteilen wie etwa Enzymen oder anorganischen Peroxidbleichmitteln zugesetzt. Die Waschmittelzusammensetzung, zu der die Aktivatorgranulate zugegeben werden, kann auf verschiedenen Wegen erhalten werden, wie etwa Trockenmischen, Extrudieren und Sprühtrocknung.

In einer weiten Ausführungsform sind die erfindungsgemäßen Bleichaktivatoren besonders geeignet für nicht wäßrige flüssige Waschmittel, zusammen mit einer bleichenden Peroxyverbindung, etwa Natriumperborat, um dem Waschmittel ein großes Reinigungsvermögen für Gewebe und Textilien zu verleihen. Derartige nicht wäßrige, flüssige Waschmittel, die pastöse und gelatinöse Detergentienzusammensetzungen mit einschließen, sind im Stand der Technik bekannt, und sind beispielsweise in US-2 864 770, US-2 940 938, US-4 772 412, US-3 368 977, GB-A-1 205 711, GB-A-1 370 377, GB-A-1 270 040, GB-A-1 292 352, GB-A-2 194 536, DE-A-2 233 771, EP-A-0 028 849 beschrieben.

Es handelt sich dabei um Zusammensetzungen in Form eines nicht wäßrigen, flüssigen Mediums, indem eine feste Phase dispergiert sein kann. Das nicht wäßrige, flüssige Medium kann eine flüssige, oberflächenaktive Substanz sein, vorzugsweise eine nichtionische oberflächenaktive Substanz; ein nicht polares flüssiges Medium, wie etwa flüssiges Paraffin; ein polares Lösungsmittel, wie etwa Polyole, zum Beispiel Glycerin, Sorbitol, Ethylenglykol, eventuell in Verbindung mit niedermolekularen einwertigen Alkoholen wie Ethanol oder Isopropanol; oder Mischungen daraus.

Die feste Phase kann aus Buildersubstanzen, Alkalien, abrasiven Stoffen, Polymeren, anderen festen ionischen oberflächenaktiven Stoffen, Bleichmitteln, fluoreszierenden Stoffen und anderen üblichen festen Inhaltsstoffen bestehen.

Es ist aus Gründen der Stabilität und Handhabbarkeit vorteilhaft, die Bleichaktivatoren in Form von Granulaten zu verwenden, die neben dem Bleichaktivator ein Bindemittel enthalten. Verschiedene Methoden, solche Granulate herzustellen, sind in der Patentliteratur beschrieben, so beispielsweise in Kanada Pat. Nr. 1 102 966, GB-1 561 333, US-4 087 369, EP-A-0 240 057, EP-A-0 241 962, EP-A-0 101 634 und EP-A-0 062 523. Jede dieser Methoden ist für die erfindungsgemäßen Bleichaktivatoren anwendbar.

Die die erfindungsgemäßen Bleichaktivatoren enthaltenden Granulate werden im allgemeinen der Waschmittelzusammensetzung zusammen mit den anderen, trockenen Bestandteilen wie etwa Enzymen, anorganischen Peroxidbleichmitteln zugesetzt. Die Waschmittelzusammensetzung, zu der die Aktivatorgranulate zugegeben werden, kann auf verschiedene Wegen erhalten werden, wie etwa Trockenmischen, Extrudieren, Sprühtrocknung.

In einer weiten Ausführungsform sind die erfindungsgemäßen Bleichaktivatoren besonders geeignet für nicht wäßrige flüssige Waschmittel, zusammen mit einer bleichenden Peroxidverbindung, etwa Natriumperborat, um dem Waschmittel ein großes Reinigungsvermögen für Gewebe und Textilien zu verleihen. Derartige nicht wäßrige, flüssige Waschmittel, die pastöse und gelatinöse Detergentienzusammensetzungen mit einschließen, sind im Stand der Technik bekannt, und sind beispielsweise in US-2 864 770, US-2 940 938, US-4 772 412, US-3 368 977, GB-A-1 205 711, GB-A-1 370 377, GB-A-1 270 040, GB-A-1 292 352, GB-A-2 194 536, DE-A-2 233 771, EP-A-0 028 849 beschrieben.

Es handelt sich dabei um Zusammensetzungen in Form eines nicht wäßrigen, flüssigen Mediums, in dem eine feste Phase dispergiert sein kann. Das nicht wäßrige, flüssige Medium kann eine flüssige, oberflächenaktive Substanz sein, vorzugsweise eine nichtionische oberflächenaktive Substanz; ein nicht polares flüssiges Medium wie etwa flüssiges Paraffin; ein polares Lösungsmittel, wie etwa Polyole, zum Beispiel Glycerin, Sorbitol, Ethylenglycol, eventuell in Verbindung mit niedermolekularen einwertigen Alkoholen, wie Ethanol oder Isopropanol; oder Mischungen daraus.

Die feste Phase kann aus Buildersubstanzen, Alkalien, abrasiven Stoffen, Polymeren, Ionen, anderen festen ionischen oberflächenaktiven Stoffen, Bleichmitteln, fluoreszierenden Stoffen und anderen üblichen festen Inhaltsstoffen bestehen.

Die folgenden, nicht abschließenden Beispiele sollen einen Überblick über die Ausführungsformen der Erfindung geben.

Beispiel 1: Synthese von Cyanamid-Dihydrochlorid

Durch eine Lösung von 21,0 g 99 %igem Cyanamid in 23 g Ethanol und 50 ml Diethylether wird über einen Zeitraum von 3 Stunden trockenes HCl-Gas eingeleitet. Durch Eiskühlung wird die Temperatur zwischen 0 und 10°C gehalten. Direkt nach Beginn der Gaseinleitung fällt ein farbloser Niederschlag aus. Nach Beendigung der Reaktion wird der Niederschlag abgesaugt. Man erhält 53,8 g Cyanamid als Dihydrochlorid (94 %).

Schmp. 180-182°C
Cl-Gehalt 60,1 %

| | | | | | |
|---|---|---|---|---|---|
| Elementaranalyse: | gef: | C 11,0; | H 3,5; | N 24,7; | Cl 60,4 |
| | ber: | C 10,4; | H 3,5; | N 24,4; | Cl 61,7 |

In analoger Weise wird die Verbindung $(CH_3)_2N$-CN * 2 HCl durch Einleiten von HCl in eine Lösung von Dimethylcyanamid in einer Mischung aus Ethanol und Dimethylether erhalten.

Beispiel 2: Synthese von N-Cyanpiperidin-Dihydrochlorid

Durch eine Lösung von 105,7 g N-Cyanpiperidin in 44 g Ethanol und 55 ml Diethylether wird über einen Zeitraum von 7 Stunden trockenes HCl-Gas eingeleitet. Durch Eiskühlung wird die Temperatur zwischen 0 und 10°C gehalten. Nach Beendigung der Reaktion wird noch 5 Stunden bei Raumtemperatur gerührt, anschließend wird das Lösungsmittel im Vakuum entfernt. Man erhält 171,4 g N-Cyanpiperidin-Dihydrochlorid (93 %).

Schmp. 180-182°C
Cl-Gehalt 37,6 %

Beispiel 3:

Durch Zusammengeben von 200 ml einer wäßrigen Lösung von 5 g/l Referenzwaschmittel (WMP), erhalten von WFK-Testgewebe GmbH, Krefeld, 150 mg Natriumperborat-Monohydrat (PB∗1) und 50 mg eines Aktivators wurde eine Bleichmittelzusammensetzung erhalten. Vier mit schwarzem Tee verschmutzte Gewebestücke (BC-1-Tee auf Baumwolle, 1,25 g, WFK) wurden für ein dreißigminütiges, isothermes Waschexperiment in einem Linitest-Gerät zugegeben. Die Gewebestücke wurden nach der vorgegebenen Waschzeit mit Wasser gespült, getrocknet, gebügelt. Anschließend wurde die Bleichwirkung mittels eines Weißgrad-Meßgerätes ELREPHO 2000 (Datacolor) festgestellt, indem die Unterschiede der Remissionen vor und nach dem Bleichen ermittelt wurden.

Das Experiment wurde mit verschiedenartigen Anschmutzungen (z.B. Gras, Curry) und bei verschiedenen Temperaturen (20°C, 40°C) wiederholt.

Vergleichsversuche wurden durchgeführt, in denen 50 mg Tetraacetylethylendiamin (TAED) der Bleichmittelzusammensetzung zugegeben wurden.

Es wurden die in Tabelle 1 angegebenen Bleichmittelzusammensetzungen mit den Bleichaktivatoren 1 bis 3 hergestellt. Ihre Wirksamkeit wurde durch den Vergleich der Remissionen des Gewebes vor und nach dem Bleichvorgang ermittelt. Die Resultate sind in Tabelle 1 angegeben. Die ΔΔR-Werte geben die Verbesserung der Bleichwirkung der erfindungsgemäßen Zusammensetzung verglichen mit PB∗1 und TAED an:

$$\Delta\Delta R\ CA{*}HX\text{-}PB{*}1 = \Delta R\ (CA{*}HX) - \Delta R\ (PB{*}1)$$

$$\Delta\Delta R\ CA{*}HX\text{-}TAED = \Delta R\ (CA{*}HX + TAED) - \Delta R\ (TAED)$$

Die Verbindungen 1 bis 3 sind

1 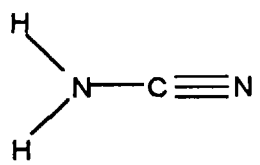 *2 HCl

2 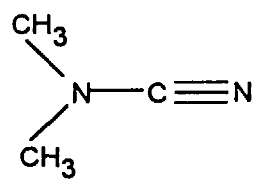 *2 HCl

3 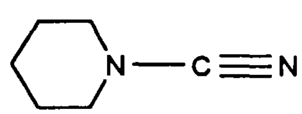 *2 HCl

Tabelle 1

| Aktivator | ΔR (CA*HX) | ΔR (CA*HX + TAED) | ΔΔR (CA*HX -PB*1) | ΔΔR (CA*HX -TAED) |
|---|---|---|---|---|
| PB∗1 | 11,5 | | | |
| TAED | | 15,1 | | |
| 1 | 16,9 | 18,0 | 5,4 | 2,9 |
| 2 | 25,6 | 25 | 14,1 | 9,9 |
| 3 | 19,1 | 17,5 | 7,6 | 2,4 |

Weitere nützliche Eigenschaften der Cyanamid-Salze sind geringe Farbschädigung und geringe Faserschädigung. Experimente mit gefärbten Fasern (Remazol® rot RB gran. 133 und Remazol® Brillantrot GG) zeigen, daß die erfindungsgemäßen Aktivatoren weitaus geringere Farbschädigungen hervorrufen als Cyanamid-Aktivatoren entsprechend dem Stand der Technik.

## Patentansprüche

**1.** Verbindungen der allgemeinen Formel

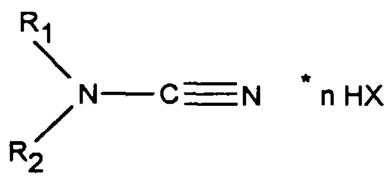

worin

$R_1$ und $R_2$ Wasserstoff, $C_1$- bis $C_{24}$-Alkyl, $C_2$-$C_{24}$-Alkenyl, Aryl, $C_1$-$C_4$-Alkylaryl oder $C_1$-$C_4$-Alkoxy-$C_1$-$C_4$-Alkyl sind, oder $R_1$ und $R_2$ zusammen mit dem Stickstoffatom, an das sie gebunden sind, einen Ring mit 4 bis 6 Kohlenstoffatomen bilden, der durch
$C_1$-$C_5$-Alkyl, $C_1$-$C_5$-Alkoxy, $C_1$-$C_5$-Alkanoyl, Phenyl, Amino, Ammonium, Cyano, Cyanamino, Chlor oder Brom substituiert sein kann, und wobei dieser Ring zusätzlich zum Stickstoff anstelle von Kohlenstoffatomen ein oder zwei Sauerstoffatome oder >NH oder >N-CN-Gruppen enthalten kann,
n 1 oder 2 ist und X ein Anion bedeutet.

**2.** Verbindungen nach Anspruch 1, dadurch gekennzeichnet, daß $R_1$ und $R_2$ unabhängig voneinander Wasserstoff, $C_1$-$C_4$-Alkyl, $C_2$-$C_4$-Alkenyl oder Phenyl bedeuten.

**3.** Verbindungen nach Anspruch 1, dadurch gekennzeichnet, daß X Chlorid, Bromid, Iodid, Fluorid, Sulfat, Hydrogensulfat, Carbonat, Hydrogencarbonat, Phosphat, Mono- und Di-Hydrogenphosphat, Pyrophosphat, Metaphosphat, Nitrat, Methosulfat, Dodecylsulfat, Dodecylbenzolsulfonat, Tosylat, Phosphonat, Methylphosphonat, Methandisulfonat, Methylsulfonat, Ethansulfonat, Formiat, Acetat oder Propionat ist.

**4.** Verbindungen nach Anspruch 1, dadurch gekennzeichnet, daß $R_1$ und $R_2$ gleich sind und Wasserstoff oder Methyl bedeuten, n = 2 ist und HX zwei Äquivalente HCl oder ein Äquivalent $H_2SO_4$ bedeuten.

**5.** Wasch- und Reinigungsmittel, enthaltend

a) 2 bis 40 Gew.-% einer Peroxyverbindung
b) 0,1 bis 20 Gew.-% einer Verbindung nach Anspruch 1 als Bleichaktivator.

6. Wasch- und Reinigungsmittel nach Anspruch 5, dadurch gekennzeichnet, daß der Bleichaktivator in einer Menge von 0,5 bis 10 Gew.-% vorhanden ist.

7. Wasch- und Reinigungsmittel nach Anspruch 5, dadurch gekennzeichnet, daß der Bleichaktivator in einer Menge von 1 bis 7,5 Gew.-% vorhanden ist.

8. Wasch- und Reinigungsmittel nach Anspruch 5, dadurch gekennzeichnet, daß die Peroxyverbindung Perborat, Percarbonat, Perphosphat, Persilikat, Monopersulfat, Harnstoffperoxid, Cumolhydroperoxid oder t-Butylhydroperoxid ist.

9. Wasch- und Reinigungsmittel nach Anspruch 5, dadurch gekennzeichnet, daß sie oberflächenaktive Substanzen in einer Menge von bis zu 50 Gew.-% enthalten.

Europäisches Patentamt

# EUROPÄISCHER RECHERCHENBERICHT

Nummer der Anmeldung
EP 97 11 1942

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US 2 768 204 A (I. HECHENBLEIKLNER)<br>* Beispiele 1,2,4-7 * | 1-4 | C07C261/04<br>C01C3/16<br>C07D295/20<br>D06L3/02<br>C11D3/39 |
| X | T.B. JOHNSON: "The nitrogen products formed by chlorination of isothioureas"<br>JOURNAL OF THE AMERICAN CHEMICAL SOCIETY,<br>Bd. 61, 1939, DC US,<br>Seiten 176-179, XP002044557<br>* Seite 177, rechte Spalte, letzter Absatz - Seite 178, linke Spalte, Absatz 1 * | 1-4 | |
| X | P. BUZEK ET AL.: "Das Reaktionsverhalten der N-Basen NH3, NC-NH2 und NC-CN gegenüber EF5 und EF5/HF (E=As, Sb)"<br>JOURNAL OF FLUORINE CHEMISTRY.,<br>Bd. 65, 1993, LAUSANNE CH,<br>Seiten 127-132, XP002044558<br>* Seite 128, rechte Spalte, letzter Absatz - Seite 129, linke Spalte, Absatz 1 * | 1-4 | |
| X | R.I. BOTTO ET AL. : "Spectrophotometric determination of uncomplexed cyanide and thiocyanate in wastewater with p-phenylenediamine"<br>ANALYTICAL CHEMISTRY.,<br>Bd. 53, 1981, COLUMBUS US,<br>Seiten 2375-2376, XP002045174<br>* Seite 2376, linke Spalte, letzter Absatz * | 1-4 | **RECHERCHIERTE SACHGEBIETE (Int.Cl.6)**<br>C07C<br>C01C<br>C07D<br>D06L<br>C11D |
| X | US 4 126 664 A (WEISS STEFAN)<br>* Spalte 1, Zeile 22 - Zeile 25 *<br>-/-- | 1-4 | |

Der vorliegende Recherchenbericht wurde für alle Patentansprüche erstellt

| Recherchenort | Abschlußdatum der Recherche | Prüfer |
|---|---|---|
| DEN HAAG | 30.Oktober 1997 | Pauwels, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 97 11 1942

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | R.D. HUNT: "FTIR Spectra of cyanamid complexes with hydrogen fluoride an solid argon"<br>J. PHYS. CHEM. ,<br>Bd. 91, 1987,<br>Seiten 2751-2755, XP002045175<br>* Seite 2753; Tabelle II *<br>--- | 1-4 | |
| X | DR. E. DRECHSEL: "Über Cyanamid"<br>J. PRAKT. CHEM.,<br>1875,<br>Seite 209 XP002045176<br>* Seite 315 *<br>--- | 1-4 | |
| A | US 4 086 177 A (KUBITSCHEK HANS E ET AL)<br>* das ganze Dokument *<br>----- | 1-9 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| DEN HAAG | 30.Oktober 1997 | Pauwels, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)